# EUROPEAN PATENT SPECIFICATION

(11) **EP 1 786 483 B1**
(45) Date of publication and mention of the grant of the patent: **03.11.2010**
(21) Application number: 05772445.2
(22) Date of filing: 10.08.2005
(51) Int. Cl.: A61L 27/32, A61L 27/54

(54) **BIOMIMETIC PROCESS FOR COATING SUBSTRATES**
BIOMIMETISCHES VERFAHREN ZUR BESCHICHTUNG VON SUBSTRATEN
PROCEDE BIOMIMETIQUE PERMETTANT DE RECOUVRIR DES SUBSTRATS

(30) Priority: 10.08.2004 NL 1026814
(43) Date of publication of application: 23.05.2007
(73) Proprietor: YEKIMED AG, CH-3067 Boll (CH)
(72) Inventor: LIU, Yuelian, NL-3512 PT Utrecht (NL)
(74) Representative: Swinkels, Bart Willem
(86) International application number: PCT/NL2005/000580
(87) International publication number: WO 2006/016807

(56) References cited:
- EP-A- 0 987 031
- US-A1- 2003 113 438
- US-B1- 6 569 489
- JONASOVA L ET AL: "Biomimetic apatite formation on chemically treated titanium" BIOMATERIALS, ELSEVIER SCIENCE PUBLISHERS BV., BARKING, GB, vol. 25, no. 7-8, March 2004 (2004-03), pages 1187-1194, XP004475062 ISSN: 0142-9612
- BARRERE F ET AL: "Nano-scale study of the nucleation and growth of calcium phosphate coating on titanium implants" BIOMATERIALS, ELSEVIER SCIENCE PUBLISHERS BV., BARKING, GB, vol. 25, no. 14, June 2004 (2004-06), pages 2901-2910, XP004489039 ISSN: 0142-9612
- DU C ET AL: "Bone growth in biomimetic apatite coated porous Polyactive<(>R) 1000PEGT70PBT30 implants" BIOMATERIALS, ELSEVIER SCIENCE PUBLISHERS BV., BARKING, GB, vol. 23, no. 23, December 2002 (2002-12), pages 4649-4656, XP004377537 ISSN: 0142-9612

## Description

### FIELD OF THE INVENTION

The present invention relates to a biomimetic process for coating substrates, particularly medical devices such as implants, to such coated substrates and to the application thereof in bone, connective tissue, fat tissue and muscle tissue engineering.

### BACKGROUND OF THE INVENTION

Research in the field of dental and orthopedic implantology is currently focused on developing tools and methodologies to potentiate osseointegration and to expedite the re-establishment of full functionality. With the realization of these aims, the healing phase and convalescence time of patients could be curtailed and their social and professional reintegration established the sooner.

An improvement in the osteoconducivity of implants has already been achieved by coating their surfaces with layers of calcium phosphate in various crystalline or amorphous forms. Attempts have also been made to render these coatings osteoinductive by the addition of osteogenic growth factors, such as transforming growth factor beta or bone morphogenetic proteins. But, until recently, this task posed a major stumbling block. Most of the techniques used to prepare inorganic coatings are performed either at extremely high temperatures (e.g., plasma spraying) or under other highly unphysiological physical conditions, which preclude the incorporation of biologically active proteinaceous molecules during their deposition.

Investigators have attempted to circumvent this difficulty by secondarily adsorbing osteogenic agents onto the surfaces of preformed inorganic layers. However, such superficially adsorbed molecules serve as only a two-dimensional, and thus limited, reservoir, which is rapidly released, in a single burst, upon exposure to a physiological environment. Hence, the osteoinductive effects of these agents are restricted both temporally and spatially. Investigators have attempted to overcome this problem by increasing the concentration of the adsorbed growth factor to unphysiological levels. However, the molecules are still released rapidly in a single burst, and the high local levels thereby generated result in undesirable, non-specific binding to collagen fibrils and other extracellular matrix molecules in the vicinity of the implant.

Preformed calcium phosphate layers have also been chemically modified in an attempt to delay the release of adsorbed growth factors. But even with such manipulations, the rate of drug release is still more rapid than from a three-dimensional (lattice-incorporated) depot. A further drawback of these physical coating techniques is that they can be applied only to highly temperature-resistant materials, such as metallic alloys, and to those with a relatively smooth surface topography.

Several methods have been proposed in recent years to deposit a coating on all kinds of substrates. These methods have been reviewed in a paper in Proc Instn Mech Engrs Vol 212 part H by K. de Groot et al. In this review paper several techniques have been described such as plasma spraying, vacuum plasma spraying, high velocity oxy-fuel spraying and further wet techniques such as electrophoretic deposition, electrochemical deposition biomimetic deposition and finally sputter techniques i.e. standard sputter deposition, ion assisted deposition, pulse laser deposition, magnetron deposition, hot isostatic pressing and frit enamelling.

Of particular interest is a biomimetic deposition method involving forming a biologically active bone like apatite layer on a substrate by immersion in a Hank's balanced salt (supersaturated) solution or simulated body fluid.

Document EP 0 987 031 describes a method for coating a substrate, particularly a medical device with a biomimetic composition comprising biologically active agents (Cf. D1, paragraph 43), D1 also describes the coated substrate.

Document US 6 569 489 describes a coated substrate and the method for coating a substrate, particularly a medical device with a biomimetic composition.

Document US 2003/00113438 describes a coated substrate, the coating comprising a bioactive substance and the method for coating said substrate, particularly a medical device with a biomimetic composition comprising biologically active agents.

US patent 6.207.218 describes a method for coating an implant comprising the steps of
a) contacting the implant with an aqueous solution of magnesium, calcium and phosphate ions;
b) passing carbon dioxide gas through the aqueous solution to maintain a concentrated solution of the ions; and
c) degassing the aqueous solution to cause precipitation of salts on the implant.

A disadvantage of this method is that the process is conducted in an open system which moreover is not maintained under sterile conditions. Moreover it is difficult to control the coating thickness and the micro conditions of the coating process. In this procedure one cannot incorporate a bioactive substance in a simple way. In order to achieve this one would need to apply a 2 steps process involving first producing a thin coating representing a seeding layer and then switching to a closed system (which may contain a bioactive molecule )and producing a secondary layer of e.g. Ca phosphate coating. Such procedure is complex and not suitable for commercial applications. It also requires a very large volume of solutions in the second step of the process which leads to very high losses of the bioactive molecules which one desires to incorporate in the coating. Therefore, the process becomes rather expensive.

### SUMMARY OF THE INVENTION

It is an object of the present invention to provide a process wherein the above mentioned disadvantages are alleviated to a great extent According to the present invention a biomimetic coating composition is produced consisting of an electrolyte solution simulating the electrolyte composition of tissues, particularly soft tissue or connective tissue, containing a bioactive substance which can be released in a sustained or delayed way, produced by coprecipitation of said bioactive material with the other components, mainly salts resulting in incorporation of the bioactive substance, preferably substantially in physiological amounts in the lattice works forming an integral part of the coating, said coating is imprinted as a film, advantageously in one step on the substrate as distinct from the 2-steps process of the prior art technique.

It is another object of the present invention to provide a coating process which is carried out in a closed system, preferably under aseptic or virtually sterile conditions.

Still another object of the present invention is to provide a process wherein the thickness of the coating and the physical state of the coating (crystalline, amorphous or mixed forms) is manipulated by pre programming the composition of the constituents of the starting mixture used.

Another object of the present invention is to provide a biomimetic process ie typically a mild process carried out at a temperature which has no detrimental effect on the activity and stability of the bioactive substance incorporated in the coating.

Still another object of the present invention is to provide a commercially viable, highly reproducible process wherein relatively low volumes are required in a mini reactor system, ie volumes less than 100 ml and preferably between 5 and 20 ml wherein the medical device or implant is soaked in the coating composition.

Finally it is an object of the present invention to provide coating compositions and devices particularly useful in connective tissue-, bone tissue- , fat tissue and muscle tissue engineering.

These and other objectives and advantages will become apparent in the more detailed description of the invention.

### DETAILED DESCRIPTION OF THE INVENTION

In its most general form the biomimetic process for coating a substrate according to claim 1 is provided.

In the process according to the invention the bioactive substance is incorporated already in the initial mixture. It coprecipitates with the inorganic salts and is scavenged in the structure or crystal lattices.

The reactor in which the process is carried out is preferably operated under aseptic or virtually sterile conditions. Ways and means for achieving this are well known in the art. For instance bacteriological filters can be used and where such is possible a heat treatment can be applied on the equipment and on solutions which can stand high temperatures around approx 100°C-110°C. Sterilisation can also be carried out using a sterilising gas. The coated substrate is subsequently air dried, or dried under an inert gas or sometimes lyophilised preferably under sterile conditions.

It is however also possible to operate the process under conditions which are not aseptic and sterilize using gamma irradiation in a later stage subsequent to step c).

The reactor is designed as a closed system. The reactor can consist of a hermetically closeable container which in its simplest form can be a glass bottle.

The process can be carried out in a mini or micro system reactor in view of the relatively low volumes (often less than 100 ml or even less than 20 ml) employed in the coating process according to the invention.

In the process according to the invention an acid is added in a quantity sufficient to dissolve all the constituents including the bioactive substance which can be a protein, taking into account the iso-electric point of said protein. It will be readily understood that in the present process acid may be added to the saline aqueous mixture or the salts may be added to acidified water. The acid used can in principle be an organic acid such as acetic acid or an anorganic acid but is preferably selected from the group consisting of hydrochloric-, sulphuric- and phosphoric acid. It is convenient to add at least part of the quantity of acid to be used to water and to subsequently add the various salts.

To enhance dissolution of all constituents of the mixture a pH ranging from 5.2-6.6 is adequate. The preferred range is 5.8-6.4.

Subsequently, the pH is allowed to raise and the mixture is stored preferably under stirring for a period sufficiently long to allow the pH to reach a value preferably ranging from 7.0-8.5 and to achieve precipitation and coating of the substrate. The increase of pH can induce the following stages: undersaturation, super saturation or a metastable state and nucleation and crystal growth. Heterogeneous nucleation takes place when a solution has reached the super saturation limit or the metastable state. At supersaturation crystals can grow from metastable solutions. At higher concentration, homogeneous nucleation or precipitation can occur. By varying the pH the above changes are modulated.

A preferred embodiment of the present invention involves producing a solution by adding the salts in the following sequence:
i) magnesium chloride, preferably in its hexahydrate form,
ii) calcium chloride, preferably in its dihydrate form,
iii) sodium hydrogen phosphate, preferably in its dihydrate form, to acidified water having a pH within the given range whereafter
iv) sodium bicarbonate is added.

After the last addition of bicarbonate the pH gradually raises to ultimately reach a value within the mild alkaline range mentioned above.

The above mentioned salts constitute the basic components stimulating the electrolyte composition of both soft and bone tissues. We have found that adding a minor amount of potassium chloride for instance 0.1-1g/l was useful when soft tissue deposits are envisioned.
The saline composition simulates the electrolyte composition of tissues .It can be advantageous in some cases to use a composition isotonic with blood.

The desired thickness of the coating is pre-programmed as it were, by a judicious selection of the components of the mixture and their respective concentrations.

A very preferred composition which has proven to be very effective is produced from: 0.2-2.0 g/l magnesium chloride, 0.4 -2.0 g/l calcium chloride, 1.0-5.0 g/l sodium bicarbonate, 0.2-1.5 g/l Na₂HPO₄.

In practice, it was found extremely useful to add an appropriate amount of sodium chloride in the composition in order to influence the nature of the coating in terms of crystallinity or amorphous state or mixed forms depending on the application envisioned. The degree of crystallinity can be monitored or measured by X ray diffraction, the so called noise pattern gives an indication of the degree of crystallinity.

Both magnesium chloride and sodium chloride reduce the speed of precipitation and slow down the precipitation process. A gradual, slow process is advantageous for getting adequate coatings of the right thickness.

Usually a concentration of sodium chloride in the aqueous mixture varying from 20-50 g/l will be adequate.

The biomimetic coating process according to the invention is usually carried out within the range 15-50 °C,preferably 20-45 °C most preferably 25-40 °Cand ideally 37C The choice of the ideal temperature depends on the nature of the bioactive substance used and the temperature at which its stability and activity could be detrimentally affected. Variation of the temperature can contribute to modulating the duration of the coating process.

The period of storage of the substrate in contact with the coating composition will usually range from 3-96 hours and preferably 5-48 hours or longer if necessary, to achieve a coating with a thickness ranging from 0.5 to 100 microns .The thickness of the coating is a factor determining the delay of release of the bioactive substance. Another factor determining the degradation time of the coating when implanted in the body is the amount of mobilised body giant cells or osteoclasts which can be triggered by incorporating suitable factors in the coating composition .Upon degradation of the coating osteogenic-,lipogenic- or connective tissue growth factors get liberated.

The obtained coating may comprise a whole variety of salts selected from calcium carbonate, dicalcium phosphate dihydrate, orthocalcium phosphate, hydroxyl carbonate apatite and the like, in amorphous, crystalline or amorphous-crystalline state and an effective amount of bioactive substance.

The substrate, medical device or implant can consist of soft or hard polymers such as collagen, polylactate gelatine, possibly in the form of a membrane, and can be bio degradable or non- biodegradable, on which is applied a sustained or delayed release coating containing, for instance, an osteogenic substance, a cell growth promoting factor such as BMP, FGF, TGF/CTGF(tissue and connective tissue growth factor), an angiogenesis factor which could be the vascular endothelial growth factor (VEGF) or FGF-2 (fibroblast growth factor-2), drugs such as an antibiotic substance, any protein, vitamin, hormone or substances inhibiting some physiological functions, which is added in the starting mixture of the components used to prepare the coating composition and coprecipitates on the substrate. It is even possible to co precipitate genes or fragments thereof which display effects similar to those of growth factors.
Interesting applications of the above principle is dentistry or plastic surgery, for instance, corrective plastic surgery on genetic defects like cleft palate.
Another interesting application relates to the induction of fat tissue formation by incorporation of an angiogenesis factor alone or in combination with a lipogenic factor for instance for achieving female breast enlargement.

Last but not least, the biomimetic compositions can be applied in a coating process involving the production of a new tooth by incorporating in the composition different signal substances for the different layers of the tooth and applying such coating on appropriate matrix-carriers,

The invention will now be illustrated in the following examples which should not be construed in a limitative way.

### EXAMPLES

### Example 1

In a closed minireactor with a volume of 20 ml a saline aqueous mixture was produced by adding while stirring the following components in water acidified with a sufficient amount of a 1M solution of HCl to reach a pH of 6.0., in the given sequence, to produce a coating composition in which the final concentrations are given between brackets.
Magnesium chloride (O.5g/l), calcium chloride (1.0g/l), Na₂HPO_{4.} (0.25g/l), NaHC0₃ (5.0g/l), sodium chloride(40g/ml).

Protein (BSA) was added as a test substance in a concentration of 20 microgram/ml.

Titanium discs of 15 mm diameter were brought into contact with the coating composition in the mini reactor. The mini reactor and its contents were stored under magnetic stirring.

During a storage period of 6 hours at room temperature. The pH raised gradually to a value around pH 8 and precipitation of salts including the protein took place, resulting in forming on the discs a film coating containing the protein whereupon the discs which were removed from the saline solution and were air dried. Coated discs were produced the thickness of which was approximately 4 microns.

All the steps of the operation were carried out at room temperature, under sterile conditions and a bacteriological filter was used to filter all solutions.

### Example 2.

Example 1 was repeated starting this time from the following mixture of salts with the given final concentrations: magnesium chloride (1.52 g/l), calcium chloride 1.84 g/l, Na₂HPO₄ (0.89 g/l), NaHCO₃ (1.76 g/l), sodium chloride (40 g/l).

Excellent coatings on the discs were obtained. The thickness of the coating was approximately 4 microns.

### Example 3.

Example 2 was repeated without sodium chloride. The coatings obtained were thinner than those obtained in example 2. The alkaline pH was reached in less than 3 hours and precipitation and coating resulted within a shorter time than in the previous example.

### Example 4.

In the following comparative experiments the following group of samples were produced and were evaluated with regard to their physical, mechanical, release properties, physiological properties both in vivo and in vitro.
a) controle uncoated sample.
b) controle sample without BMP-2 protein.
c) controle sample with BMP-2 protein using a preformed layer and superficially absorbed BMP.
d) representative sample according to the invention involving coprecipitation of BMP-2 protein and salts.

Investigated was the osteoinductive potential of titanium-alloy implants bearing BMP-2-containing biomimetic coatings in vivo. For this purpose, a well established ectopic (subcutaneous) ossification model in rats was used, which is widely employed to screen the osteogenic activity of biomaterials and bioactive agents . For the first time using this model, data relating to the net weekly rate of bone formation and to the rate of coating degradation (which is probably cell-mediated) during the course of a 5-week follow-up period were presented. The spatial extent of BMP-2-induced bone formation is also quantified. This parameter is of practical clinical importance in the bridging of large gaps, which frequently fail to heal after surgical implantation. Furthermore, under local osteoporotic conditions, the active and rapid induction of peri-implant bone formation may help to improve the mechanical stability of prostheses at an early stage.

### Materials and methods

### Study design

This study is divided into two parts: the first consists of in vitro experiments relating to the preparation and characterization of implant coatings; the second consists of the in vivo implantation experiments in rats, including a histological and histochemical analysis and a histomorphometrical evaluation of bone tissue formation and coating degradation during the course of a 5-week follow-up period.

### In vitro experiments

### Preparation of implant coatings

Titanium-alloy (Ti6A14V) discs (1 cm in diameter) were immersed in 5 times concentrated simulated body fluid (MgCl2 1.52g/l,CaCl2 1.84g/l Na2HPo4 0.89g/l,NaCl 40g/l;NaHCO3 1.76g/l,for 24 h at 37°C under high-nucleation conditions to inhibit crystal growth. The fine, dense layer of amorphous mixture of salts as described in example 1 thereby produced serves as a seeding surface for the deposition of a crystalline layer. The latter was produced by immersing samples (n = 90) in a supersaturated solution of mixture of salts as described in example 1 (ph 7.4), which either lacked (n = 60) or contained (n = 30) human recombinant BMP-2 (10 mg/l) Wyeth, Cambridge, MA, USA), for 40 h at 37°C. As a positive control for superficially adsorbed BMP-2, a portion (n = 30) of the mixture of salts as described in example 1 coatings prepared in the absence of BMP-2 were then immersed in phosphate-buffered saline (PBS) containing BMP-2 (10 mg/l) for 48 h at 37°C. All samples were prepared under sterile conditions and then stored at -80C.

### Quantification of BMP-2 associated with coatings

The amount of BMP-2 associated with each coating was determined by ELISA (n = 6 for each variant (10)). Each coating was removed mechanically from its underlying titanium-alloy disc and dissolved in 1 ml of 20% EDTA. Samples of the extract were diluted 10 or 100 times and 100-µl aliquots of each were then transferred to 96-well microplates. 100 -µl of anti-human recombinant BMP-2 [(Wyeth, Cambridge, MA, USA) diluted 1:10000 in PBS] were added to each well and the intensity of the colored reaction product was measured colorimetrically at an absorption wavelength of 405 nm using a microplate reader. Readings were converted into ng of BMP-2 using a calibration curve.

### Measurement of coating thicknesses

The initial thickness of each coating was measured in vitro using a magnetic induction probe (Electrophysik minitest 2100, Germany), the measuring range of which lay between 0 and 100 µm. Six measurements were taken for each sample. and the average value was determined.

### Fourier-transform infrared spectroscopy

This teçhnique was used to determine the crystalline features of the coatings. Each coating was peeled away from its underlying titanium-alloy disc and 0.6-.0.8 mg of the sample material was mixed with 280 mg of potassium bromide. The resulting transparent pellet was analyzed in a Fourier-transform infrared spectrometer (spectrum 1000, Perkin-Elmer. UK).

### Scanning electron microscopy and energy-dispersive X-ray analysis

Coated titanium-alloy discs were sputtered with carbon particles to a thickness of 12-16 mm. They were then examined in a scanning electron microscope (model 525, Philips, Eindhoven;. The Netherlands) and simultaneously subjected to an energy-dispersive X-ray analysis (EDX.
Voyager. Philips. Eindhoven, The Netherlands).

### Mechanical strength of coatings

The mechanical strength of each coating was assessed by means of a micro-scratch test, which was performed using an advanced mechanical surface-testing system (CSEM Instruments, Neuchatel. Switzerland). It involved generating a scratch with a spherical diamond stylus (Rockwell C diamond; tip diameter: 100 µm). which was drawn at a constant speed (10 mm per minute) across the coating (still attached to its underlying titanium-alloy disc) under progressively increasing loads, produced at a constant rate (30 N per minute). The critical load, namely, that at which scratching generates not a "clean" cut but disintegrated (non-coherent) material, depends (among other factors) upon the mechanical strength (adhesion and cohesion) of the coating.

### In vivo experiments

### Experimental design

One experimental (incorporated BMP-2) and three control groups were set up: titanium-alloy discs bearing a coprecipitated layer of mixture of salts as described in example 1 and BMP-2 ( incorporated BMP-2 group);
naked titanium-alloy discs [negative control for the effects of a calcium phosphate layer and of BMP-2 (uncoated group)] titanium-alloy discs bearing a biomimetic layer of mixture of salts as described in example 1 only [negative control for the effects of BMP.2 (no-BMP-2 group)]; and titanium-alloy discs bearing a biomimetic layer of mixture of salts as described in example 1 and superficially adsorbed BMP-2 {positive control for BMP-2 (adsorbed-BMP-2 group )]. Six discs per group and per time point were implanted subcutaneously in rats. Each animal received two discs, one on the left side and one on the right side, at a dorsal site. The discs on contralateral sides of any given rat were in all animals derived from different test groups. However, each rat always received either BMP-2-containing discs (incorporated-BMP-2 group or adsorbed-BMP-2 group) or non-BMP-2-containing ones (uncoated group or no-BMP-2 group). This strategy was adopted to avoid the possibility of cross-reactivity. With this precondition, the various disc types were distributed among the 60 animals according to a systematic protocol. In a preliminary study, no cross-reactivity occurred between discs in the incorporated BMP-2 group and those in the adsorbed-BMP-2 group (i.e., an osteogenic response was observed in the former case but not in the latter). The implanted discs were retrieved for analysis at 7-day interval over a period of 5 weeks (see Table 1).

### Implantation

Sixty young adult male Wistar rats (weighing 185-250 g) were used for this study. The animals were fed a standard diet and had unlimited access to water. Surgery was performed under conditions of general anesthesia [using Vetalar ® (ketamine hydrochloride)). The left and right dorsal regions of each rat were shaved and disinfected and the skin was incised. One implant was inserted subcutaneously on each side of each animal and the surgical cut was then closed by suturing. The animals were housed in compliance with the Dutch guidelines for animal experimentation and the study was approved by the Dutch animal experimental study committee (Cbe/00/13883).

### Explanation, tissue processing and sampling protocol

Rats were killed by administering an overdose of gaseous carbon dioxide. The implants were retrieved, together with a minimum quantity of surrounding tissue, by sharp dissection. This minimum was determined by the degree of implant encapsulation with connective tissue. Material was fixed by immersion in 10% formaldehyde at ambient temperature for several days. Samples were then rinsed in tap water, dehydrated in ethanol and embedded in methylmethacrylate. Applying a systematic random sampling protocol [22], five slices, each 600 µm in thickness and 2 mm apart, were prepared from each sample using a diamond saw. The slices were mounted on plexiglass holders, polished and surface-stained with McNeil's Tetrachrome, basic Fuchsine and Toluidine Blue O [46] in preparation for histological analysis in the light microscope.

### Histomorphometrical evaluation

Bone formation, coating degradation and resorptive cell activity (coverage by foreign body giant cells or osteoclasts) were evaluated histomorphometrically. Using a defined systematic random sampling protocol at two different final magnifications ( x 74 and x 184), eight digital images per section (i.e., for each of the five sections taken per disc) were obtained in a Nikon-Eclipse light microscope and printed in color. The histomorphometrical analysis was performed on these colored prints using the point- and intersection counting methodologies elaborated by Cruz-Orive et al Gunderson et al. described in the literature.. The following morphometric parameters were determined; the volume density of bone tissue per section per time point, and the volume density of coating material present were estimated using Cavalieri's method described in the literature. The net rate of bone formation per disc per week and the net rate of coating degradation per disc per week were then calculated per group for each postoperative week.

The maximal distance away from the implant surface at which the neoformation of bone was observed on each section was measured perpendicular to this surface using a ruler. The mean maximal distance was then determined for each group at each time point (when applicable).

### Histochemical staining for TRAP

Using sections that were surface-stained with McNeil's Tetrachrome, basic Fuchsine and Toluidine Blue O, the percentage of the implant or coating surface covered with multinucleated cells (i.e., foreign body giant cells plus osteoclasts) was estimated by intersection counting, using a line system that was oriented perpendicular to the disc surface. After completion of this and the other morphometrical analyses described in the previous section, the tissue specimens were polished down by approximately 20-30 µm for histochemical staining according to the tartrat-resistant acid phosphatase (TRAP) reaction using a standard protocol. Only osteoclasts are TRAP-positive; foreign body giant cells remain unstained. Using the same intersection counting technique as that described above, the percentage of the implant or coating surface covered with TRAP-positive cells (i.e. osteoclasts) was estimated. The percentage of the surface covered with foreign body giant cells was determined by subtracting the number of TRAP-positive cells (i.e., osteoclasts) from the total number of multinucleated cells (estimated using conventionally stained sections).

### Statistical analyses

Differences between the various groups at a particular sampling time (surface coverage with foreign body giant cells in each of the four groups; coating volumes in the three relevant groups), and within the same group at each sampling time, were statistically analysed using the one way ANOVA test, the level of significance being set at P < 0.05. SAS statistical software (version 8.2) was employed for this purpose. Post hoc comparisons were then made using Bonferroni corrections. Data pertaining to coating thickness, the total amount of BMP-2 incorporated into coatings, the total amount of BMP-2 adsorbed onto coatings, coating strength (micro-scratch test), the total volume of bone deposited per time point to the incorporated BMP-2 group, bone coverage of discs per time point in the incorporated BMP-2 group, and surface coverage with osteoclasts per time point in the incorporated BMP-2 group, were analysed using the same statistical tests. All numerical data are presented as mean values together with either the standard deviation (SD) or the standard error of the mean (SEM).

### Results

### In vitro data

### Incorporation of BMP-2 into biomimetic coatings

Coatings prepared by the co-precipitation of mixture of salts as described in example 1 and BMP-2 were revealed by ELISA to have incorporated 1.7 ± 0.079 µg (mean ± SD) of the osteogenic growth factor per disc, or 0.5 ± 0.138 µg per mg of coating. The amount of BMP-2 adsorbed superficially upon the surfaces of preformed mixtures of salts as described in example 1 was significantly lower (P < 0.05) at 0.98 was significantly lower (P < 0.05) at 0.98 ± 0.045 µg (mean ± SD) per disc, or 0.1 ± 0.0003 µg of coating (Table 1).

### Coating characteristics

Coatings were of uniform thickness, with mean (± SD) values of 25.00 (± 6.4) µm (n = 6) and 23.85 (± 4.8) µm (n = 6) for those prepared in the absence and presence of BMP-2, respectively. There was no significant difference between the two. Preformed mixtures of salts as described in example 1 bearing a layer of superficially adsorbed BMP-2 did not differ significantly in thickness before and after the deposition of this agent.

Fourier transform infrared spectroscopy revealed biomimetic coatings to possess the features typical of an octacalcium phosphate crystal structure, irrespective of the absence or presence of BMP-2. Scanning electron microscopy disclosed the inorganic latticework to be composed entirely of straight, plate-like crystal units with sharp edges; no change in this geometry was elicited by the presence of BMP-2.

The micro-scratch test revealed mixtures of salts as described in example 1 prepared in the presence of BMP-2 to possess greater mechanical strength than those prepared in the absence of this agent, the critical lead in each case, namely, that at which scratching generated not a "clean" cut but disintegrated (non-coherent) material, being 2.5 ± 0.37 N (mean ± SD) and 1.8 ± 0.08 N (mean ± SD), respectively (P < 0.05; n = 6 for each group).

### In vivo data

### Histology

After the first week of implantation, discs in the uncoated group and in the no-BMP-2 group were covered with foreign body giant cells to an areal extent of 80-90%. Coverage decreased progressively during the ensuing 4 weeks. By the end of the fifth week, coverage in the uncoated group (41%) was significantly higher ( P < 0.05) than in any of the three coated groups (Fig. 1 and Table 2).

In the uncoated group and in the no-BMP-2 group , a mild inflammatory response involving lymphocytes and macrophages was observed up to distances of 50-200 µm from the discs. The degree and extent of this response decreased gradually with time. The discs underwent progressive encapsulation with vascularized connective tissue. By the fifth week, large needle-like crystalline structures were apparent at many locations along discs in the no-BMP-2 group. These structures bore no resemblance to those constituting the original inorganic latticework. No such deposits were observed in the uncoated group.

Discs in the adsorbed BMP-2 group were likewise covered with foreign body giant cells to an areal extent of 80% after the first week of implantation (Fig. 1), and mild inflammatory responses were similarly observed within the immediate surroundings. Close to the discs, small islands of woven bone with adhering osteoclasts and osteoblasts were very occasionally observed. But this osteogenic activity was so rare as to be non-measurable morphometrically. It was based upon a-direct, not an enchodral, ossification mechanism. After the second week of implantation, these islands of bone tissue had been completely resorbed. During the remainder of the follow-up course, no further evidence of osteogenic activity was manifested, either along the coating surface or within the surrounding connective tissue. The mild inflammatory response apparent during the early postoperative phase (weeks 1 and 2) had virtually ceased by the third week. At this latter juncture, a substantial connective tissue capsule was observed. Areal coverage of the coatings with foreign body giant cells had decreased to 60% by the third week and to 25% by the fifth (Fig. 1). At this latter juncture, discs in the adsorbed BMP-2 group bore deposits of needle-like crystalline structures which were similar to those observed in the no BMP-2 group.

In the incorporated BMP-2 group, the histological picture manifested after the first week of implantation correspond to that recorded for the uncoated group and the no B MP-2 group. No osteogenic activity was apparent. By the second week, many osteogenic foci were seen, not only upon the coatings but also at distances of up to 150 ± 5.3 (SEM) µm therefrom. This osteogenic activity was based overwhelmingly upon a direct ossification mechanism. Only a few sites of enchondral bone formation were observed. Indeed, these were so rare as to be non-measurable morphometrically. Osteogenic activity was sustained during the third, fourth and fifth weeks (Figs. 4a-d) leading to the formation of an increasing mass of woven bone. Bone trabeculae were observed both in direct contact with the coatings and within the connective tissue capsule. Bone narrow tissue was apparent not only between the bone trabeculae but also in direct contact with the coatings. By the fifth week, the mild inflammatory response was almost completely quelled, but the resorption of coatings by foreign body giant cells (and osteoclasts) continued. Foreign body giant cells often occupied portions of the coatings that were not covered with bone. At 5 weeks, the areal coverage of coatings with foreign body giant cells (11%) was lower in this incorporated BMP-2 group than in any of the others (Fig. 1). However, if the number of foreign body giant cells was expressed relative to the disc area that was devoid of newly-formed bone or osteoclasts, then the areal coverage was similar in each of the three coated groups [24-32% )data not presented)]. By the end of the fifth week, discs in the incorporated BMP-2 group were almost completely surrounded by woven bone. Bone tissue was observed at maximal distances of 340 ± 13 (SEM) µm and 217 ± 5.4 (SEM) µm from the discs at the third and fifth weeks, respectively. The decrease in this distance is probably attributable to enhanced bone remodelling (see next section: Histomorphometry).

In each group, native tissue reactivity to the disc surfaces was surprisingly low. Apart from connective tissue encapsulation, the inflammatory responses were mild and abated with time. They were never observed to spread beyond a distance of 400 µm from the implant surface. No signs of acute rejection, such as the presence of granulocytes, were ever apparent.

### Histomorphometry

Histomorphometry revealed no measurable evidence of osteogenic activity during the first week of implantation in any of the groups. After the second week, bone tissue was deposited around discs in the incorporated BMP-2 group, but in none of the others. The net volume of bone formed increased from 5.8 mm³ at the second week to 10.3 mm³ at the third. By the fourth week, it had decreased to 6.8 mm³, but then increased again to around the third week value at 5 weeks [10.4 mm³. The net weekly rate of bone formation was maximal during the second week (5.8 mm³ per disc per week); it dropped slightly during the third (4.49 mm³ per disc per week), and again during the fifth week (3.64 mm³ per disc per week). During the fourth week, the net weekly rate of bone resorption exceeded that of bone formation. Nevertheless, bone coverage of the implants increased steadily between the third and fifth weeks. By the enc of the fifth week most of the discs in the incorporated BMP-2 group were completely surrounded by newly-formed woven bone. At this latter juncture, the coating volume has decreased by about 60% [calculated on the basis of the "zero" time-point value . Hence, during the 5-week follow-up period, bone mass increased and coating volume decreased.

Disc coatings in the BMP-2 group and in the incorporated BMP-2 group were degraded irregularly during the 5-week follow-up period. Those in the adsorbed BMP-2 group underwent degradation only during the third (substantial) and fifth weeks (Table 3).

### TRAP Histochemistry

Histochemical staining for TRAP revealed the multinucleated population of cells associated with discs in each of the three control groups to be exclusively TRAP-negative. They were thus all identified as foreign body giant cells. After the first week of implantation, discs in the incorporated BMP-2 (experimental) group were covered only with TRAP-negative (foreign body giant) cells. But after the second week, when osteogenic activity became manifest, TRAP-positive cells (i.e., osteoclasts) appeared. The presence of both foreign body giant cells and osteoclasts probably accounts for the high rates of coating degradation observed during the third and fifth weeks (Table 3) in this group.

### Discussion

The osteoconductivity of metallic implants used in dentistry and orthopaedic surgery can be enhanced by coating their surfaces with a layer of either a mixture of salts as described in example 1 based or bone matrix-like material These inorganic layers are of course three-dimensional latticeworks, which can be used to deliver osteoinductive agents to the peri-implant site. In a previous study, we incorporated the osteogenic growth factor BMP-2 into mixtures of salts as described in example 1 using the biomimetic technique. BMP-2 formed an integral part of the three-dimensional inorganic latticework and was not merely adsorbed upon its surface . Furthermore, the osteogenicity of BMP-2 thus incorporated was not only retained but also potentiated in an in vitro system comprised of cultured osteoprogenitor cells .

In the present study, we wished to evaluate the osteoinductive potential of titanium-alloy implants bearing a biomimetically co-precipitated layer of BMP-2 and mixtures of salts as described in example 1 in vivo, using an ectopic (subcutaneous) rat model.

Our histological and histomorphometrical findings confirmed our expectations: BMP-2 incorporated into biomimetic coatings not only induced ectopic bone formation at a very low pharmacological level, but sustained this process over the entire 5-week follow-up period. When BMP-2 was only superficially adsorbed onto preformed biomimetic coatings, it was unable to induce more than a very transient and sporadic osteogenic response, which endured no longer than 1week and which was quantitatively negligible, even though the total amount of BMP-2 deposited exclusively on the surface (0.98 µg per disc) did not differ greatly from that distributed throughout the entire latticework in the incorporated BMP-2 group (1.7 µg per disc).

That bone tissue was laid down by a direct rather than by an enchondral mechanism was an unexpected finding of our study. In other investigations that have made use of this ectopic ossification rat model, BMP-2 induced an enchondral ossification cascade, which was sustained for no longer than 12-14 days; after this time, bone resorption began and was complete by the third week. Direct ossification is known to occur only within a mechanically stable field, in the absence of shear stress. And in our system, such an environment was obviously furnished by the rigid titanium-alloy discs. In previous studies, BMP-2 was bound either to small particles or to collagenous or glass matrices , which are subject to frictional contact during the movement of a rat's skin muscles. In only one study was BMP-2 applied within a large membranous mass of fibrillar collagen, and in this instance, direct ossification took place.

In our study, unlike the situation in these previous investigations, bone tissue did not begin to resorb after 2 weeks,. It was formed continually during the remaining 3 weeks of the follow-up period. A decrease in the weekly rate of bone formation was observed during the fourth wee, but this reflected the predominance of bone remodelling activities at this stage, which is to be expected in the absence of active stress fields. And despite this circumstance, the total volume of bone present at the fourth week was not substantially lowered. Furthermore, the loss was confined to the implant surroundings, since bone coverage of the discs themselves increased steadily (without a dip) between the third and fifth weeks . After 5 weeks, approximately 40% of the coating material remained undergraded, which suggests that a similar portion of the incorporated BMP-2 was unliberated. The implication is that osteogenic activity could have continued for several more weeks after the termination of the experiment. The sustainment of MP-2 delivery and osteogenic activity is of course the purpose of an osteoinductive coating; and this property is of great importance for the optimal osseointegration of an implant. Since approximately 60% of the coating material was degraded during the 5-week follow-up period, 60% of the initially incorporated amount of BMP-2 (1.7 µg per disc) was probably also released during this period, that is, 1.02 µg during the course of 5 weeks. That this amount of BMP-2 was sufficient to induce and sustain osteogenic activity at a relatively high level throughout the 5 weeks, whereas a similar quantity of superficially adsorbed BMP-2 (0.98 µg per disc) elicited no more than a very transient, sporadic and abortive osteogenic response when released in a single burst of short duration (probably not exceeding a few days), implies that a lower bit sustained pharmacological level of the drug is osteogenically more potent and efficient than a higher dose delivered over a short timespan.

Other types of application system for the controlled release of growth factors have also been described in the literature . For example, poly-D.L-lactide has been used to carry insulin-like growth factor-1 and transforming growth factor beta-1. In combination with this drugcarrier, these osteogenic agents were likewise more efficient in stimulating osteogenesis at a low but sustained dose rate than they were when administered freely in a single high dose (analogous to burst release from a superficially adsorbed depot).

The osteoinductive efficacy of BMP-2 has been tested also in other systems. For example it has been applied directly to mixtures of salts as described in example 1 coated collagen matrices and to cement . However, the concentration of BMP-2 that was required to elicit an osteogenic response was several orders of magnitude higher than that used in the present study. Indeed, we have ourselves shown that when BMP-2 is delivered to an ectopic site in rats via collagen sponges, a higher concentration of the drug is required to induce osteogenic activity than when it is incorporated biomimetically into mixtures of salts as described in example 1 . These findings emphasize the importance of the mode of drug delivery. When less biocompatible materials are used to carry BMP-2, this agent has a lower bioactivity, owing to the high level of adverse tissue reactivity (i.e. an augmented foreign body giant cell response). Likewise in conjunction with such materials, BMP-2 elicits a very early and intense bone resorption reaction, which could dominate over bone formation activities.

During the first postoperative week, the degradation of biomimetic coatings containing in mixture of salts as described in example 1 corporated BMP-2 was mediated exclusively by foreign body giant cells; only thereafter did osteoclast participate in the process . The high rate of coating degradation observed during and after the third week (Table 3), which led to a significant reduction in coating volume by the end of the fifth week, is most probably accounted for by the synergistic resorptive activities of the foreign body giant cells and osteoclasts. During the initial post-surgical phase (the first week), foreign body giant cells, in being drawn to the site of implantation as part of the inflammatory response mounted against foreign material, and in embarking on their destructive tasks by attacking the coating, may actually promote osteogenic activity by liberating BMP-2 from the inorganic matrix as they degrade it. They could thus assume the role played by osteoclast in physiological bone formation and in remodelling-based signalling path-ways a role which the osteoclasts themselves fulfil in our model after the first week of implantation. Hence, the potentially destructive foreign body giant cells could function in a constructive capacity. However, we have at present no evidence to support this hypothesis. It could of course be argued that BMP-2 is released spontaneously from the coatings. However, in a previous study, the spontaneous release of an incorporated model protein (bovine serum albumin) was found to be negligible according to the ELISA assay . The quantity of BMP-2 liberated in this manner is probably also negligible and almost certainly leis below the osteoinductive threshold. This surmise is supported by our findings for the adsorbed BMP-2 group. These coatings induced only an abortive osteogenic response during the first postoperative week, and the islands of bone formed were so small and so rare as to be quantitatively non-measurable; by the second week, this osseous tissue had been completely resorbed.

Bone tissue was deposited not only in the immediate vicinity of discs in the incorporated BMP-2 group, but also directly upon their surfaces. Bone marrow, too, was observed in direct contact with these coatings. These findings, particularly the latter, indicate that the biomimetic coatings were highly biocompatible, since bone marrow contains immunocompetent cells which are very sensitive to foreign material. That the surface areal coverage of these coatings with foreign body giant cells was only moderate at the end of the 5-week follow-up period also argues in this direction.

Osteogenic foci were also observed within the connective tissue capsules of discs in the incorporated BMP-2 group. Hence, collagen fibrils therein apparently served as a stable framework for direct bone growth. Bone tissue was deposited at a distance of 340 ± 13 (SEM) µm from the discs after 3 weeks. This is an impressive finding for an ectopic site, which differs both biochemically and mechanically from the orthotopic one. The sustained local levels of BMP-2 may have accounted for this phenomenon. By the end of the fifth week, this maximal distance had decreased to 217 ± 5.4 (SEM) µm, indicating that the initially widespread bone-formation process was becoming circumscribed to the immediate vicinity of the implant and that bone remodelling activities were augmented. Albeit so, osteogenic activity still predominated at this final juncture, and the total bone mass was still increasing.

In summary, the incorporation of BMP-2 into biomimetic coatings according to the invention yields highly biocompatible, osteoconductive and osteoinductive properties. Furthermore, BMP-2 is released not only at a level that suffices to induce osteogenesis, but also gradually, most likely in a cell-mediated manner, such that osteogenic activity is sustained for a considerable period of time. In future experiments, this principle will be optimized for application at orthopic sites.

## Claims

1. A process for coating a substrate, particularly a medical device, with a biomimetic composition , which comprises:
a) producing batchwise in a closed system an acidified biomimetic composition comprising a saline aqueous mixture containing calcium-, magnesium-, phosphate-, bicarbonate ions and a bioactive substance,
b) contacting the substrate with said biomimetic composition and
c) storing the biomimetic composition in contact with the substrate and allowing a gradual increase of the pH to occur and to cause precipitation of salts and co-precipitation of the bioactive substance on said substrate and to obtain a coated substrate, said bioactive substance being selected from the group consisting of an osteogenic growth factor, a cell growth promoting factor, an angiogenesis factor, a lipogenic factor, an antibiotic substance, a protein, a vitamin, a hormone, an inhibitor, a gene or gene fragment displaying effects similar to those of growth factors.

2. A process according to claim 1,wherein coprecipitation and imprinting of a coating film on the substrate is achieved in one single step.

3. A process according to claim 1, wherein the biomimetic composition is passed through a bacteriological filter before being contacted with the substrate.

4. A process according to claim 1 , wherein all salts and bioactive substance in the saline solution are completely dissolved and which saline solution has a pH ranging from 5.2-6.6, which is produced by adding an acid.

5. A process according to anyone of the preceding claims, wherein the pH is adjusted by adding an acid selected from the group consisting of hydrochloric acid, sulphuric acid, phosphoric acid and combinations thereof.

6. A process according to any one of the preceding claims, wherein the biomimetic composition is stored in contact with the substrate until the pH has increased to a value ranging from 7.0-8.5.

7. A process according to anyone of the preceding claims, wherein the biomimetic composition is prepared by adding to acidified water
i) magnesium chloride,
ii) calcium chloride, iii) sodium hydrogen phosphate,
whereafter
iv) sodium bicarbonate is added and the pH is allowed to increase.

8. A process according to claim 7, wherein the salts are added and dissolved in acidified water in the given sequence i), ii), iii), iv)

9. A process according to claims 7-8, wherein the following salts are incorporated in the biomimetic composition in the indicated concentrations:
0.2-2.0 g/l magnesium chloride, 0.4 -2.0 g/l calcium chloride, 1.0-5.0 g/l sodium bicarbonate, 0.2-1.5 g/l Na₂HPO₄ and optionally 0.1-1g/l potassium chloride.

10. A process according to anyone of the preceding claims wherein the period of storage of the substrate in contact with the coating composition ranges from 5-48 hours and the thickness of the resulting coating is within the range of 0.5-100 microns.

11. A process according to claims 1-10 wherein the composition further contains sodium chloride.

12. A process according to claim 10, wherein sodium chloride is used in a concentration of 20-50g/l.

13. A process according to anyone of the preceding claims 1-12,wherein a biomimetic composition is produced for soft tissue deposits by including a potassium salt in an amount preferably ranging from 0.1-1g/l.

14. A process according to anyone of the preceding claims wherein the saline composition in the initial mixture is isotonic with blood.

15. A process according to anyone of the preceding claims, yielding a coating comprising calcium carbonate, dicalcium phosphate dihydrate, orthocalcium phosphate and hydroxyl carbonate apatite, in amorphous, crystalline or amorphous-crystalline state or mixed forms thereof, and an effective amount of bioactive substance.

16. A process according to anyone of the preceding claims wherein the substrate comprises a soft or hard polymer selected from the group consisting of collagen, polylactate and gelatine.

17. A process according to anyone of the preceding claims, wherein the substrate is a bone prosthesis, a tooth prosthesis or a breast prosthesis.

## Patentansprüche

1. Verfahren zur Beschichtung eines Substrates, insbesondere einer medizinischen Vorrichtung mit einer biomimetischen Zusammensetzung, umfassend:
a) Batch-weises Erzeugen einer angesäuerten biomimetischen Zusammensetzung in einem geschlossenen System, welche in salzhaltiges wässriges Gemisch aus Calcium-, Magnesium-, Phosphat-, Bicarbonat-Ionen und eine bioaktive Substanz umfasst,
b) In-Kontakt-Bringen des Substrates mit der biomimetischen Zusammensetzung, und
c) Lagern der biomimetischen Zusammensetzung in Kontakt mit dem Substrat und Ermöglichen eines graduellen Anstiegs des pH, damit eine Präzipitation der Salze und eine Co-Präzipitation der bioaktiven Substanz auf dem Substrat stattfindet und ausgelöst wird, und um ein beschichtetes Substrat zu erhalten, wobei die bioaktive Substanz ausgewählt ist aus der Gruppe bestehend aus einem osteogenen Wachstumsfaktor, einem Zellwachstumfsförderungsfaktor, einem Angiogenesefaktor, einem lipogenen Faktor, einer antibiotischen Substanz, einem Protein, einem Vitamin, einem Hormon, einem Inhibitor, einem Gen oder Genfragment, das ähnliche Wirkungen zeigt wie die der Wachstumsfaktoren.

2. Verfahren nach Anspruch 1, wobei die Co-Präzipitation und das Aufbringen des Beschichtungsfilmes auf dem Substrat in einem einzigen Schritt durchgeführt werden.

3. Verfahren nach Anspruch 1, wobei die biomimetische Zusammensetzung vor dem Kontakt mit dem Substrat über einen bakteriologischen Filter durchgeleitet wird.

4. Verfahren nach Anspruch 1, wobei sämtliche Salze und die bioaktive Substanz in der salzhaltigen Lösung vollständig gelöst sind und wobei die salzhaltige Lösung einen pH im Bereich von 5,2 bis 6,6 aufweist, welcher durch das Hinzufügen einer Säure hergestellt wird.

5. Verfahren nach einem der vorhergehenden Ansprüche, wobei der pH durch Hinzufügen einer Säure eingestellt wird, welche ausgewählt ist aus der Gruppe bestehend aus Salzsäure, Schwefelsäure, Phosphorsäure und Kombinationen davon.

6. Verfahren nach einem der vorhergehenden Ansprüche, wobei die biomimetische Zusammensetzung in Kontakt mit dem Substrat gelagert wird, bis der pH auf einen Wert im Bereich von 7,0 bis 8,5 angestiegen ist.

7. Verfahren nach einem der vorhergehenden Ansprüche, wobei die biomimetische Zusammensetzung hergestellt wird, indem zu angesäuertem Wasser
i) Magenisumchlorid,
ii) Calciumchlorid,
iii) Natriumhydrogenphosphat hinzugefügt wird, und danach
iv) Natriumbicarbonat
hinzugefügt wird und der pH ansteigen gelassen wird.

8. Verfahren nach Anspruch 7, wobei die Salze zu dem angesäuerten Wasser in der vorgegebenen Reihenfolge i), ii), iii), iv) hinzugefügt und gelöst werden.

9. Verfahren nach einem der Ansprüche 7 bis 8, wobei die folgenden Salze in der biomimetischen Zusammensetzung mit den vorgegebenen Konzentrationen enthalten sind:
0,2 - 2,0 g/l Magnesiumchlorid, 0,4 - 2,0 g/l Calciumchlorid, 1,0 - 5,0 g/l Natriumbicarbonat, 0,2 - 1,5 g/l Na₂HPO₄ und gegebenenfalls 0,1 - 1 g/l Kaliumchlorid.

10. Verfahren nach einem der vorhergehenden Ansprüche, wobei die Lagerungszeit des Substrates in Kontakt mit der beschichteten Zusammensetzung im Bereich von 5 bis 48 Stunden liegt und die Stärke der entstehenden Beschichtung in einem Bereich von 0,5 bis 100 Mikrometer liegt.

11. Verfahren nach einem der Ansprüche 1 bis 10, wobei die Zusammensetzung ferner Natriumchlorid enthält.

12. Verfahren nach Anspruch 10, wobei Natirumchlorid in einer Konzentration von 20 bis 50 g/l eingesetzt wird.

13. Verfahren nach einem der vorhergehenden Ansprüche 1 bis 12, wobei eine biomimetische Zusammensetzung für Weichgewebeablagerungen hergestellt wird, indem ein Kaliumsalz in einer Menge in einem Bereich von vorzugsweise 0,1 bis 1 g/l hinzugefügt wird.

14. Verfahren nach einem der vorhergehenden Ansprüche, wobei die salzhaltige Zusammensetzung in dem Anfangsgemisch mit Blut isotonisch ist.

15. Verfahren nach einem der vorhergehenden Ansprüche, wobei es zu einer Beschichtung kommt, welche Calciumcarbonat, Dicalciumphosphatdihydrat, Orthocalciumphosphat und Hydroxylcarbonatapatit in amorphem, kristallinem oder amorphkristallinem Zustand oder Mischformen davon sowie eine wirksame Menge der bioaktiven Substanz umfasst.

16. Verfahren nach einem der vorhergehenden Ansprüche, wobei das Substrat ein Weich- oder Hartpolymer, ausgewählt aus der Gruppe bestehend aus Kollagen, Polylactat und Gelatine umfasst.

17. Verfahren nach einem der vorhergehenden Ansprüche, wobei das Substrat eine Knochenprothese, eine Zahnprothese oder eine Brustprothese ist.

## Revendications

1. Procédé de revêtement d'un substrat, en particulier d'un dispositif médical avec une composition biomimétique comprenant les étapes consistant à :
a. préparer de façon discontinue, dans un système clos, une composition biomimétique acidifiée renfermant un mélange salin aqueux contenant des ions calcium, magnésium, phosphate, bicarbonate et une substance bioactive,
b. mettre en contact le substrat avec cette composition biomimétique, et
c. conserver la composition biomimétique en contact avec le substrat et permettre une augmentation graduelle du pH pour provoquer la précipitation de sels et la co-précipitation de la substance bioactive sur le substrat, et pour obtenir un substrat revêtu, la substance bioactive étant choisie dans le groupe formé par un facteur de croissance ostéogénique, un facteur de promotion de la croissance cellulaire, un facteur angiogénique, un facteur lipogénique, une substance antibiotique, une protéine, une vitamine, une hormone, un inhibiteur, un gène ou un fragment de gène présentant des effets similaire à ceux des facteurs de croissance.

2. Procédé conforme à la revendication 1, selon lequel on effectue la co-précipitation et l'impression d'un film de revêtement sur le substrat, en une seule étape.

3. Procédé conforme à la revendication 1, selon lequel on fait passer la composition biomimétique au travers d'un filtre bactériologique avant sa mise en contact avec le substrat.

4. Procédé conforme à la revendication 1, selon lequel tous les sels et la substance bioactive en solution saline sont totalement dissous, la solution saline ayant un pH situé dans la plage 5,2-6,6 qui est obtenu par addition d'un acide.

5. Procédé conforme à l'une quelconque des revendications précédentes, selon lequel on règle le pH par addition d'un acide choisi dans le groupe formé par l'acide chlorhydrique, l'acide sulfurique, l'acide phosphorique et les combinaisons de ces acides.

6. Procédé conforme à l'une quelconque des revendications précédentes, selon lequel on conserve la composition biomimétique en contact avec le substrat jusqu'à ce que le pH ait augmenté jusqu'à une valeur comprise dans la plage de 7,0-8,5.

7. Procédé conforme à l'une quelconque des revendications précédentes, selon lequel on prépare la préparation biomimétique en ajoutant à de l'eau acidifiée :
i. du chlorure de magnésium,
ii. du chlorure de calcium,
iii. de l'hydrogènophosphate de sodium, puis
iv. on ajoute du bicarbonate de sodium et on laisse le pH augmenter.

8. Procédé conforme à la revendication 7, selon lequel on ajoute les sels et on les dissout dans l'eau acidifiée selon les séquences i), ii), iii), iv).

9. Procédé conforme aux revendications 7-8, selon lequel on incorpore les sels suivants dans la composition biomimétique, avec les concentrations suivantes :
0,2-2,0 g/l de chlorure de magnésium, 0,4-2,0 g/l de chlorure de calcium, 1,0-5,0 g/l de bicarbonate de sodium, 0,2-1,5 g/l de Na₂HPO₄, et le cas échéant 0,1-1 g/l de chlorure de potassium.

10. Procédé conforme à l'une quelconque des revendications précédentes, selon lequel la durée de conservation du substrat en contact avec la composition de revêtement est comprise entre 5 et 48 heures, et l'épaisseur du revêtement résultant est compris dans la plage de 0,5-100 microns.

11. Procédé conforme aux revendications 1-10, selon lequel la composition renferme en outre du chlorure de sodium.

12. Procédé conforme à la revendication 10, selon lequel on utilise le chlorure de sodium à une concentration de 20-50 g/l.

13. Procédé conforme à l'une quelconque des revendications précédentes 1-12, selon lequel on prépare une composition biomimétique destinée à être déposée sur un tissu mou en ajoutant un sel de potassium en une quantité de préférence comprise dans la plage de 0,1-1 g/l.

14. Procédé conforme à l'une quelconque des revendications précédentes, selon lequel la composition saline présente dans le mélange initial est isotonique par rapport au sang.

15. Procédé conforme à l'une quelconque des revendications précédentes, permettant d'obtenir un revêtement renfermant du carbonate de calcium, du phosphate dicalcique di-hydraté, du phosphate de calcium ortho et de l'hydroxyapatite, carbonatée dans un état amorphe, cristallin ou amorphe-cristallin, ou des formes mixtes de ces composés, et une quantité efficace d'une substance bioactive.

16. Procédé conforme à l'une quelconque des revendications précédentes, selon lequel le substrat renferme un polymère mou ou dur choisi dans le groupe formé par le collagène, le poly-lactate et la gélatine.

17. Procédé conforme à l'une quelconque des revendications précédentes, selon lequel le substrat est une prothèse d'os, une prothèse de dent ou une prothèse de sein.
